(19) Europäisches Patentamt
European Patent Office
Office européen des brevets

(11) Veröffentlichungsnummer: **0 168 035 B1**

(12) # EUROPÄISCHE PATENTSCHRIFT

(45) Veröffentlichungstag der Patentschrift: **09.10.91**

(51) Int. Cl.[5]: **C12Q 1/28**

(21) Anmeldenummer: **85108525.8**

(22) Anmeldetag: **09.07.85**

Die Akte enthält technische Angaben, die nach dem Eingang der Anmeldung eingereicht wurden und die nicht in dieser Patentschrift enthalten sind.

(54) **Verwendung von Anilinderivaten als Kupplungskomponente in Oxidativen Farbbildungsreaktionen.**

(30) Priorität: **09.07.84 DE 3425219**

(43) Veröffentlichungstag der Anmeldung:
**15.01.86 Patentblatt 86/03**

(45) Bekanntmachung des Hinweises auf die Patenterteilung:
**09.10.91 Patentblatt 91/41**

(84) Benannte Vertragsstaaten:
**AT BE CH DE FR GB IT LI LU NL SE**

(56) Entgegenhaltungen:
**EP-A- 0 161 482**
**BE-A- 649 682**
**DE-A- 3 037 342**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Batz, Hans-Georg, Dr.**
**Traubinger-Strasse 63**
**W-8132 Tutzing(DE)**
Erfinder: **Herrmann, Rupert, Dr.**
**Heinrichstrasse 3**
**W-8120 Weilheim(DE)**
Erfinder: **Topfmeier, Fritz, Dr.**
**Zeppelinstrasse 157**
**W-6900 Heidelberg(DE)**
Erfinder: **Schlumberger, Helmut**
**Kaiser-Heinrich-Strasse 23**
**W-8121 Polling(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**W-8000 München 86(DE)**

**EP 0 168 035 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Verwendung von Anilinderivaten als Kupplungskomponenten in oxidativen Farbbildungsreaktionen.

Die oxidative Farbkupplung (Emerson-Trinder-Reaktion) von Phenolen oder Anilinen mit geeigneten Kupplungspartnern, wie z. B. mit 4-Aminoantipyrin (4-AAP) oder mit Methylbenzthiazolon-hydrazon (MBTH)-kann als Nachweis und zur Bestimmung des für die Farbkupplung verwendeten Oxidationsmittels dienen. Diese Reaktion kann als Grundlage für chemische Diagnostizierverfahren in der enzymatischen Analyse oder auch auf anderen Gebieten, wie z. B. in der Immunologie, herangezogen werden, z. B. zur Bestimmung von Glukose, Harnsäure oder Cholesterin in Körperflüssigkeiten durch Bestimmung des Wasserstoffperoxids, das bei der Oxidation dieser Stoffe mit Enzymen, wie Glukoseoxidase, Urikase und Cholinesterase, gebildet wird, zur Substrat- und/oder Peroxidasen-Bestimmung mit Wasserstoffperoxid als Oxidationsmittel oder aber zur Bestimmung von Peroxiden, wie z. B. Lipidperoxiden in Körperflüssigkeiten (vgl. z. B. DE-OS 30 37 342). Eine wesentliche Voraussetzung für derartige Bestimmungsmethoden ist eine hohe Empfindlichkeit, d. h. die Bildung von Farbstoffen mit einem hohen Extinktionskoeffizienten und in hoher Ausbeute. Dies ist z. B. insbesondere in der klinisch-chemischen Diagnostik bei der Bestimmung von Substanzen, die nur in geringen Mengen in Körperflüssigkeiten vorkommen, wichtig, da hierbei Störungen durch z. B. Serumbestandteile, weitgehend ausgeschlossen werden können; ebenso wie in der klinisch-chemischen Diagnostik besteht aber auch auf anderen Gebieten, z. B. in der Immunologie, wo oft Peroxidase als Markerenzym eingesetzt wird, ein Bedarf an empfindlichen chromogenen Systemen.

Es ist bekannt, daß man bei der oxidativen Farbkupplung von Phenolen mit zur Farbbildung geeigneten Kupplungspartnern, wie z. B. mit 4-Aminoantipyrin (4-AAP) oder Methylbenzthiazolonhydrazon (MBTH) die - auf die Oxidationsmittelmenge bezogene - Farbausbeute erhöhen kann, wenn man in 4-Stellung chlorierte oder bromierte Phenole in die Kupplungsreaktion einsetzt. Dieser Effekt beruht darauf, daß es sich bei der Farbbildungsreaktion mit halogenierten Phenolen um einen 2-Elektronen-Oxidationsprozeß handelt, während die in 4-Stellung unsubstituierten Phenole in einem 4-Elektronen-Oxidationsprozeß in die farbigen Verbindungen übergeführt werden (vgl. z. B. die japanische Patentpublikation 9821/79). Die Empfindlichkeit bei der Bestimmung von Oxidationsmitteln, wie z. B. $Fe^{3+}$ oder enzymatisch gebildetem $H_2O_2$, wird also verdoppelt, wenn man 4-Halogenphenole bei der Farbbildungsreaktion verwendet.

Im Gegensatz zu den Halogenphenolen zeigen Anilinderivate, die in 4-Stellung mit Chlor oder Brom substituiert sind, bei der oxidativen Farbkupplung mit 4-AAP oder MBTH keine oder nur eine geringe Farbbildung (vgl. J. Org. Chem. 3 (1938) 153; Analytical Chemistry 33 (1961) 722); während die mit Wasserstoff substituierten aber sonst analogen Verbindungen, in der Regel, insbesondere im neutralen bis schwach sauren pH-Bereich, Farbstoffe mit höheren Extinktionskoeffizienten und längerwelligen Absorptionsmaxima als Phenole liefern. Auch mit N-substituierten Anilinen, die in o- oder p-Stellung durch eine Niederalkylgruppe substituiert sind, werden hinsichtlich Empfindlichkeit und Farbstabilität keine zufriedenstellenden Ergebnisse erzielt (vgl. DE-AS 28 33 612).

Es hat deshalb nicht an Versuchen gefehlt, neue empfindliche chromogene Systeme zur Bestimmung von Oxidationsmitteln, wie z. B. von Wasserstoffperoxid und Peroxidase (POD), zu entwickeln. Bei der oxidativen Farbkupplung führten die Entwicklungen zu verbesserten anilinischen Kupplungskomponenten, wobei die Empfindlichkeitssteigerung dabei meist durch Variation der Substituenten am anilinischen Stickstoff oder am C-3-Atom erzielt wurde (vgl. z. B. DE-OS 30 37 342; DE-OS 28 33 612; EP-A-0007787).

Aufgabe der vorliegenden Erfindung ist deshalb die Bereitstellung neuer Anilinderivate, die bei der oxidativen Farbkupplung mit hoher Ausbeute und Selektivität zu. Farbstoffen mit hoher Extinktion und Farbstabilität führen, und die deshalb ein empfindliches chromogenes System bei der oxidativen Farbkupplung bilden. Diese Aufgabe wird mit der vorliegenden Erfindung gelöst.

Gegenstand der Erfindung ist die Verwendung von Anilinderivaten der Formel I

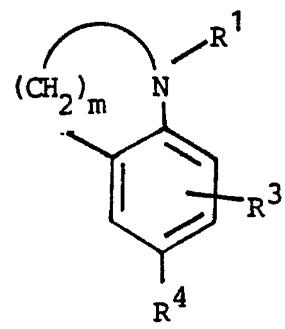

(I)

worin $R^1$ = H oder $(CH_2)_n$-X, worin n = eine ganze Zahl von 1 bis 3, X = H, OH, $NH_2$, $CH_3CONH$, $CH_3SO_2NH$, COOH, $SO_3H$ oder $ArSO_3H$, worin Ar = gegebenenfalls substituierter Arylenrest, m eine ganze Zahl von 2 bis 4 und vorzugsweise 3 ist, $R^3$ = H, Alkyl mit 1 bis 3 Kohlenstoffatomen, $OCH_3$, $CH_3CONH$, $CO_2H$ oder $SO_3H$, und $R^4$ = H, Cl, Br, COOH oder $SO_3H$, als Kupplungskomponente in oxidativen Farbbildungsreaktionen.

Der Rest Ar kann substituiert oder vorzugsweise unsubstituiert sein und ist z. B. 1,4-Naphthylen und insbesondere 1,4-Phenylen; ein substituierter Arylenrest kann einen oder mehrere, vorzugsweise einen oder zwei Substituenten besitzen, wie z. B. Alkyl mit 1 bis 3 Kohlenstoffatomen, OH, $OCH_3$, $SO_3H$ und/oder Halogen, wie insbesondere Chlor.

Eine Alkylgruppe mit l bis 3 Kohlenstoffatomen in der Bedeutung von $R^3$ ist eine Methyl-, Äthyl-, Propyl- oder Isopropylgruppe.

Besonders bevorzugt sind Anilinderivate der allgemeinen Formel II

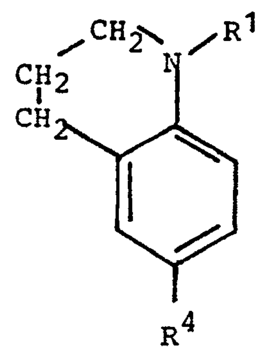

(II)

worin $R^1$ und $R^4$ die oben für die Formel I angegebenen Bedeutungen besitzen, und $R^1$ insbesondere $(CH_2)_n$-X ist, worin n 2 oder 3 bedeutet, und X = OH, $NH_2$, $CH_3CONH$, $CH_3SO_2NH$, COOH und $R^4$ insbesondere H ist.

Es wurde gefunden, daß die Spektren der bei der oxidativen Kupplungsreaktion der Anilinderivate der Formel I mit Farbkupplern entstehenden Farbstoffe überraschenderweise höhere $\epsilon$-Werte sowie sehr breite Extinktionsmaxima zeigen; letztere verringern die Abhängigkeit von einer ganz bestimmten Wellenlänge (vgl. z. B. die Fig. l bis 3). Darüber hinaus zeigen die erfindungsgemäßen Anilinderivate der Formel I im Vergleich zu den entsprechenden nicht-cyclischen N-Alkyl- oder N,N-Dialkylverbindungen überraschenderweise auch eine wesentlich bessere Farbstabilität und einen niedrigeren Leerwertschleich bei der oxidativen Kupplungsreaktion mit Farbkupplern, z. B. mit Methylbenzthiazolonhydrazon (MBTH) oder sulfoniertem Methylenbenzthiazolonhydrazon (SMBTH), wodurch eine höhere Selektivität zu erhalten ist.

Die durch polare Gruppen substituierten Verbindungen der allgemeinen Formel I zeigen auch eine verbesserte und zum Teil, insbesondere mit Alkylsulfonsäure- oder Sulfonsäuregruppen, sogar gute Wasserlöslichkeit; durch Verwendung geeigneter üblicher Detergentien können aber auch die weniger löslichen Verbindungen der Formel I in eine beständige Lösung überführt werden.

In der nachfolgenden Tabelle I werden die bei $\lambda$ max gemessenen Extinktionskoeffizienten ($cm^2/\mu mol$ $H_2O_2$) von mit $H_2O_2$/POD oxidierten chromogenen Systeme, die gebräuchliche anilinische Kupplungskomponenten und einen Farbkuppler enthalten, und die Extinktionskoeffizienten sonst gleicher chromogener

Systeme, die aber anstelle der gebräuchlichen anilinischen Kupplungskomponenten ein erfindungsgemäßes entsprechend substituiertes Anilinderivat der Formel I enthalten, gegenübergestellt. Als Farbkuppler wurde sulfoniertes Methylbenzthiazolonhydrazon (SMBTH) eingesetzt.

Die Kupplungsreaktion erfolgt auf an sich bekannte Weise (vgl. z.B. DE-AS 28 33 612); vorzugsweise wird bei Raumtemperatur gearbeitet, wobei sich ein Konzentrationsverhältnis von Kupplungskomponente/Farbkuppler > 5 als besonders zweckmäßig erwiesen hat. Die pH-Werte liegen vorzugsweise im neutralen Bereich. Der Tabelle I liegen die folgenden Werte zugrunde (Konzentrationen im Reaktionsgemisch):

Konzentrationen im Reaktionsgemisch:

| | |
|---|---|
| Kalium-Phosphat-Puffer, pH 7,0 : | 0,1 mol/l |
| anilinische Kupplungskomponente: | $3.10^{-1}$ mmol/l |
| Farbkuppler (SMBTH): | $3.10^{-2}$ mmol/l |
| Wasserstoffperoxid: | $1,5.10^{-2}$ mmol/l |
| Peroxidase: | 1,2 U/ml |

Tabelle I

| Kupplungskomponente | $\lambda_{max}$ (nm) | $\varepsilon$ ($cm^2/\mu mol\ H_2O_2$) |
|---|---|---|
| EMAE[1)] | 581 | 20,8 |
| EHT[2)] | 573 | 17,0 |
| EST[3)] | 584 | 19,2 |
| <u>Verbindungen der Formel I:</u> | | |
| $R^1 = CH_2-CH_2-NH-CO-CH_3$, m=3<br>$R^3 = 7-CH_3$, $R^4 = H$ | 557 | 27,2 |
| <u>Verbindungen der Formel II:</u> | | |
| $R^1 = CH_2-CH_2-NH-CO-CH_3$, $R^4 = H$ | 581 | 30,7 |
| $R^1 = CH_2-CH_2-OH$, $R^4 = H$ | 571 | 30,5 |
| $R^1 = (CH_2)_3-SO_3H$, $R^4 = H$ | 573 | 31,1 |

Tabelle I (Fortsetzung)

1) N-(2-Acetamidoäthyl)-N-äthyl-3-methyl-anilin

2) N-Äthyl-N-(2-hydroxyäthyl)-3-methyl-anilin

3) N-Äthyl-N-(3-methylphenyl)-2-aminoäthan-sulfonsäure

Aus der Tabelle I ist ersichtlich, daß mit den erfindungsgemäß verwendeten Anilinderivaten unter sonst gleichen Bedingungen wesentlich höhere Extinktionswerte (um den Faktor 1,3 bis 1,8) erhalten werden; mit den erfindungsgemäßen Verbindungen werden somit Kupplungskomponenten bereitgestellt, deren Verwendung zusammen mit üblichen Farbkupplern ein sehr empfindliches chromogenes System für die oxidative Farbkupplung ergibt. Die Verbindungen der allgemeinen Formel I werden insbesondere zur Bestimmung von $H_2O_2$ und POD verwendet.

Gegenstand der Erfindung ist auch ein Mittel zur analytischen Bestimmung von oxidierenden Substanzen, insbesondere $H_2O_2$, durch oxidative Farbkupplung, das dadurch gekennzeichnet ist, daß es als Kupplungskomponente eine erfindungsgemäße Verbindung der Formel I oder Vorzugsweise II enthält.

Als Farbkuppler können die für solche Farbkupplungsreaktionen gebräuchlichen Farbkuppler eingesetzt werden, wie z.B. sulfoniertes Methylbenzothiazolonhydrazon (SMBTH).

Die bei der oxidativen Farbkupplung aus den erfindungsgemäß verwendeten Kupplungskomponenten und dem Farbkuppler erhaltenen Farbstoffe lassen sich leicht zu den entsprechenden Leukofarbstoffen reduzieren, die wieder reoxidiert werden können und daher ebenfalls für den Nachweis oxidierender Substanzen, wie z. B. $H_2O_2$ in Körperflüssigkeiten, verwendet werden können.

Die Verbindungen der Formel I sind bekannt oder lassen sich nach an sich bekannten Synthesewegen herstellen, z.B. durch Alkylierung von Anilinderivaten der Formel I, worin $R^1$ = H, mit Verbindungen der Formel $Cl-(CH_2)_n-X$, worin X und n die oben angegebene Bedeutung besitzen (vgl. z.B. Houben-Weyl, Methoden der Organischen Chemie, Band II/I, Kap. II und III; Band 2/2, Seite 25I).

Die als Ausgangsmaterialien dienenden Anilinderivate der Formel I mit $R^1$ = H lassen sich z. B. durch katalytische Hydrierung der entsprechenden Chinolinderivate erhalten (vgl. Houben-Weyl, Band 4/Ic, Seite 27I).

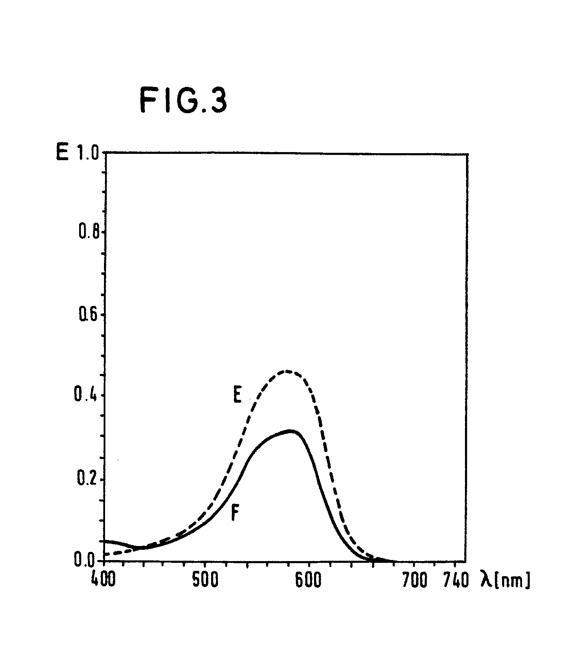

Die Fig. 1 bis 3 zeigen die Spektren der chromogenen Systeme der in Tabelle I aufgezählten Kupplungskomponenten mit sulfoniertem Methylbenzthiazolonhydrazon (SMBTH) als Farbkuppler bei der Farbbildung durch Oxidation mit $H_2O_2$/POD. Die Konzentration im Reaktionsgemisch entspricht den für Tabelle I angegebenen Werten.

In den Figuren bedeuten die Kurven A bis F die mit folgenden Kupplungskomponenten erhaltenen Spektren, in denen die Extinktion $\epsilon$ gegen die Wellenlänge $\lambda$(nm) aufgetragen ist:

A: Formel II, $R^1$ = $CH_2CH_2OH$, $R^4$ = H
B: EHT
C: Formel II, $R^1$ = $CH_2CH_2CH_2SO_3H$, $R^4$ = H
D: EST
E: Formel II, $R^1$ = $CH_2CH_2NHCOCH_3$, $R^4$ = H
F: EMAE.

Ein Vergleich der Spektren zeigt, daß die mit den erfindungsgemäß verwendeten Verbindungen erhaltenen Spektren im Vergleich zu denen mit ähnlich substituierten gebräuchlichen anilinischen Kupplungskomponenten insbesondere wesentlich höhere $\epsilon$-Werte und ein breiteres Maximum aufweisen.

## Beispiele

### Beispiel 1: N(2-Hydroxyäthyl)-1,2,3,4-tetrahydrochinolin

13,3 g (0,1 Mol) 1,2,3,4-Tetrahydrochinolin und 16,1 g (0,2 Mol) 2-Chloräthanol werden 4 Stunden auf 120 °C erhitzt. Nach dem Abkühlen wird das Reaktionsgemisch mit 200 ml Wasser versetzt und mit 2 N Natronlauge alkalisch gestellt. Anschließend wird zweimal mit Chloroform extrahiert, die organische Phase über Natriumsulfat getrocknet und am Rotationsverdampfer eingedampft. Der Rückstand wird säulenchromatographisch an Kieselgel (Elution mit Xylol/Methyläthylketon = 1/1) gereinigt.
Ausbeute: 10,5 g = 59 % d. Th.

Massenspektrum: $M^+$ = 177.

Auf analoge Weise wird durch Umsetzung von 1,2,3,4-Tetrahydrochinolin mit 2-Acetamido-propylchlorid das N-(2-Acetamidopropyl)-l,2,3,4-tetrahydrochinolin erhalten.

Beispiel 2: l,2,3,4-Tetrahydrochinolin-N-propansulfonsäure

In einem 500 ml-Kolben wurden zu 58,4 g l,2,3,4-Tetrahydrochinolin 48,8 g geschmolzenes l,3-Propansulfon bei 60 bis 80 °C innerhalb von 50 Minuten zugetropft. Das Reaktionsgemisch wurde 3,45 Stunden bei ca. 70 °C belassen, und anschließend unter Rückfluß portionsweise 470 ml Methanol zugegeben und bis zum vollständigen Lösen des festen, glasartigen Bodenkörpers unter Rückfluß weitergerührt. Danach wurde unter Rühren auf Raumtemperatur abgekühlt, wobei eine rasche Kristallisation erfolgte. Nach ca. 12-stündigem Stehenlassen im Kühlschrank wurden die Kristalle abfiltriert. Ausbeute: ca. 50 g, Schmelzpunkt: 233-237 °C.

Beispiel 3: 6-Brom-1,2,3,4-tetrahydrochinolin-N-propansulfonsäure

a) Bromierung von 1,2,3,4-Tetrahydrochinolin:

Zu einer Lösung von 13,3 g 1,2,3,4-Tetrahydrochinolin in 60 ml Eisessig wurden bei 10 bis 15 °C (Kühlung mit Eiswasser) innerhalb von 50 Minuten unter intensivem Rühren 16 g Brom, in 70 ml Eisessig gelöst, zugetropft. Der gebildete Kristallbrei wurde mit 3 x 30 ml Eisessig verdünnt, weitere 10 - 15 Minuten gerührt, 200 ml gesättigte Natriumacetatlösung und danach 300 ml Äther zugegeben. Nach Verdünnen mit Wasser auf 2 l wurde die Ätherphase abgetrennt, 2 x mit Wasser gewaschen, mit $Na_2SO_4$ getrocknet und bei 30 °C im Vakuum eingedampft. Es wurden 19,3 g eines braunen, öligen Rückstands erhalten. Der Rückstand wurde der Säulenchromatographie unterworfen (Kieselgel; Diisopropyläther/Chloroform/Eisessig). Aus den ersten substanzhaltigen Fraktionen (Fraktionen 1 - 8, je 15 ml) wurden 6,23 g 5,6-Dibrom-1,2,3,4-tetrahydrochinolin erhalten, aus den letzten Fraktionen (16 - 28, je 15 ml) 3,75 g 6-Brom-1,2,3,4-tetrahydrochinolin, Schmelzpunkt: 30 bis 34 °C.

b) 6-Brom-1,2,3,4-tetrahydrochinolin-N-propansulfonsäure

4,2 g des nach a) erhaltenen 6-Brom-1,2,3,4-tetrahydrochinolins wurden in 20 ml Aceton gelöst und unter Rühren bei 50 °C innerhalb von 30 Minuten 2,44 g geschmolzenes 1,3-Propansulfon,in 20 ml Aceton gelöst, zutropfen gelassen. Nach 24-stündigem Rückflußkochen wurden nochmals 2,44 g geschmolzenes Propansulfon, in 20 ml Aceton gelöst, unter den gleichen Bedingungen wie oben zutropfen gelassen. Nach 48-stündigem Kochen unter Rückfluß wurde die Reaktionsmischung einer Säulenchromatographie unterworfen ($SiO_2$, Elutionsmittel $CHCl_3$/Methanol = 1/1, 25 ml-Fraktionen); die Fraktionen 6-10 wurden bei 35 °C im Vakuum eingedampft, der erhaltene rotbraune Rückstand (1,8 g) mit ca. 20 ml Methanol verrührt und 1,05 g sandfarbene Kristalle erhalten; Schmelzpunkt: 217 °C (Zers.).

Auf analoge Weise läßt sich aus dem nach a) erhaltenen 5,6-Dibrom-1,2,3,4-tetrahydrochinolin die 5,6-Dibrom-1,2,3,4-tetrahydrochinolin-N-propansulfonsäure erhalten.

Beispiel 4: N-(2-Acetamidoäthyl)-l,2,3,4-tetrahydrochinolin

l3,3 g (0,l mol) l,2,3,4-Tetrahydrochinolin und 9,4 g (0,ll mol) Acetylaziridin in 200 ml Methanol (abs.) werden über Nacht am Rückfluß gekocht. Das nach dem Eindampfen am Rotationsverdampfer erhaltene Gemisch aus nichtumgesetztem Ausgangsmaterial und Reaktionsprodukt (l3 g) wird durch Säulenchromatographie an Kieselgel (Laufmittel: Chloroform/Aceton = 4 : l) gereinigt. Schmelzpunkt: l02 °C.

Beispiel 5: N-(2-Acetamidoäthyl)-7-methyl-l,2,3,4-tetrahydrochinolin

4 g (27 mmol) 7-Methyl-l,2,3,4-tetrahydrochinolin und 2,5 g (30 mmol) N-Acetylaziridin in l00 ml absolutem Äthanol werden eine Stunde am Rückfluß gekocht und anschließend im Vakuum eingedampft. Durch Säulenchromatographie an Kieselgel (Laufmittel Chloroform/Aceton = 4 : l) und Umkristallisation aus Diisopropyläther wird das Reaktionsprodukt gereinigt. Ausbeute: l,3 g; Schmelzpunkt: 99 °C.

**Patentansprüche**

1. Verwendung von Anilinderivaten der Formel I

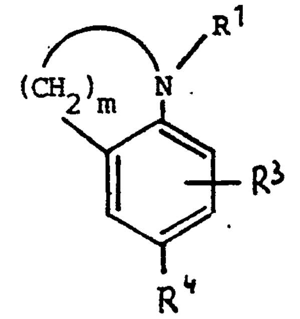

(I)

worin $R^1$ = H oder $(CH_2)_n$-X, worin n = eine ganze Zahl von 1 bis 3, X = H, OH, $NH_2$, $CH_3CONH$, $CH_3SO_2NH$, COOH, $SO_3H$ oder $ArSO_3H$, worin Ar = gegebenenfalls substituierter Arylenrest, m eine ganze Zahl von 2 bis 4 ist, $R^3$ = H, Alkyl mit 1 bis 3 Kohlenstoffatomen, $OCH_3$, $CH_3CONH$, $CO_2H$ oder $SO_3H$, und $R^4$ = H, Cl, Br, COOH oder $SO_3H$, als Kupplungskomponente in oxidativen Farbbildungsreaktionen.

2. Verwendung nach Anspruch 1, dadurch gekennzeichnet, daß man Anilinderivate der Formel II verwendet

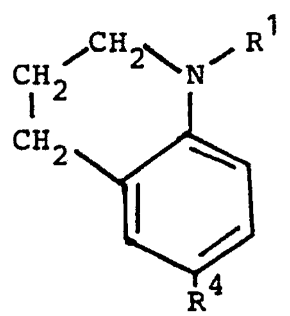

(II)

worin $R^1$ = $(CH_2)_n$-X, n = 2 oder 3, und X = OH, $NH_2$, $CH_3CONH$, $CH_3SO_2NH$, COOH oder $SO_3H$, und $R^4$ = H, Cl, Br, COOH oder $SO_3H$.

3. Verwendung nach Anspruch 2, dadurch gekennzeichnet, daß man Anilinderivate der Formel II verwendet, worin $R^1$ die in Anspruch 2 angegebene Bedeutung besitzt und $R^4$ = H.

4. Verwendung nach einem der Ansprüche 1 bis 3 zur Bestimmung von Wasserstoffperoxid und POD.

5. Mittel zur analytischen Bestimmung von oxidierenden Substanzen, insbesondere Wasserstoffperoxid, durch oxidative Farbkupplung,
**dadurch gekennzeichnet,**
daß es als Kupplungskomponente eine Verbindung der Formel I oder II nach einem der Ansprüche 1 bis 3 und einen Farbkuppler enthält.

## Claims

1. Use of aniline derivatives of the formula I

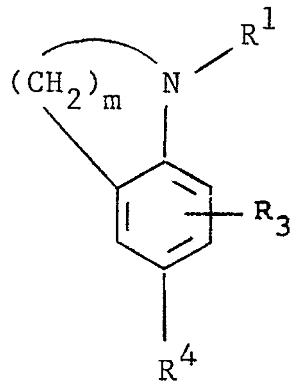

(I)

wherein $R^1$ = H or $-(CH_2)_n-X$, wherein n = a whole number of 1 to 3, X = H, OH, $NH_2$, $CH_3CONH$, $CH_3SO_2NH$, COOH, $SO_3H$ or $-ArSO_3H$, wherein Ar = possibly substituted arylene radical, m is a whole number of 2 to 4, $R^3$ = H, alkyl with 1 to 3 carbon atoms, $OCH_3$, $CH_3CONH$, $CO_2H$ or $SO_3H$ and $R^4$ = H, Cl, Br, COOH or $SO_3H$, as coupling components in oxidative colour formation reactions.

**2.** Use according to claim 1, characterised in that one uses aniline derivatives of the formula II

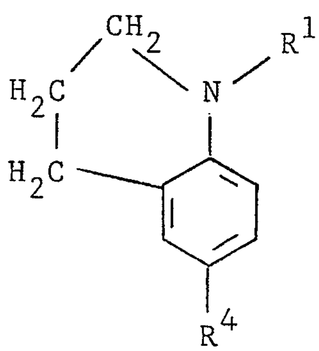

(II)

wherein $R^1$ = $-(CH_2)_n-X$, n = 2 or 3 and X = OH, $NH_2$, $CH_3CONH$, $CH_3SO_2NH$, COOH or $SO_3H$ and $R^4$ = H, Cl, Br, COOH or $SO_3H$.

**3.** Use according to claim 2, characterised in that one uses aniline derivatives of the formula **II**, wherein $R^1$ possesses the meaning given in claim 2 and $R^4$ = H.

**4.** Use according to one of claims 1 to 3 for the determination of hydrogen peroxide and POD.

**5.** Agent for the analytical determination of oxidising substances, especially of hydrogen peroxide, by oxidative colour coupling, characterised in that, as coupling component, it contains a compound of the formula I or II according to one of claims 1 to 3 and a colour coupler.

## Revendications

**1.** Utilisation de dérivés de l'aniline de formule I

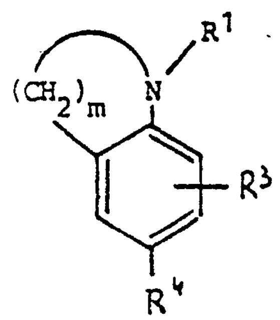

(I)

dans laquelle $R^1$ = H ou $(CH_2)_n$-X, n étant un nombre entier de 1 à 3, X = H, OH, $NH_2$, $CH_3CONH$, $CH_3SO_2NH$, COOH, $SO_3H$ ou $ArSO_3H$, Ar étant un reste arylène éventuellement substitué, m étant un nombre entier de 2 à 4, $R^3$ = H, un groupe alkyle de 1 à 3 atomes de carbone, $OCH_3$, $CH_3CONH$, $CO_2H$ ou $SO_3H$, et $R^4$ = H, Cl, Br, COOH ou $SO_3H$, comme composants de copulation dans des réactions de formation oxydante de colorant.

2. Utilisation selon la revendication 1, caractérisée en ce que l'on utilise des dérivés de l'aniline de formule générale II

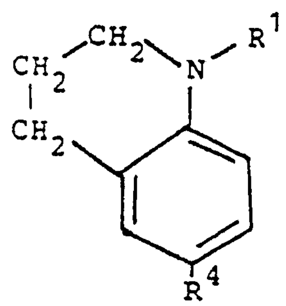

(II)

dans laquelle $R^1$ = $(CH_2)_n$-X, n est égal à 2 ou 3, et X = OH, $NH_2$, $CH_3CONH$, $CH_3SO_2NH$, COOH ou $SO_3H$, et $R^4$ = H, Cl, Br, COOH ou $SO_3H$.

3. Utilisation selon la revendication 2, caractérisée en ce que l'on utilise des dérivés de l'aniline de formule II dans laquelle $R^1$ a la signification donnée dans la revendication 2 et $R^4$ = H.

4. Utilisation selon l'une des revendications 1 à 3 pour la détermination du peroxyde d'hydrogène et de POD.

5. Agent pour la détermination analytique de substances oxydantes, en particulier du peroxyde d'hydrogène, par copulation colorée oxydante, caractérisé en ce qu'il contient comme composant de copulation un composé de formule I ou II selon l'une des revendications 1 à 3 et un coupleur coloré.

FIG.1

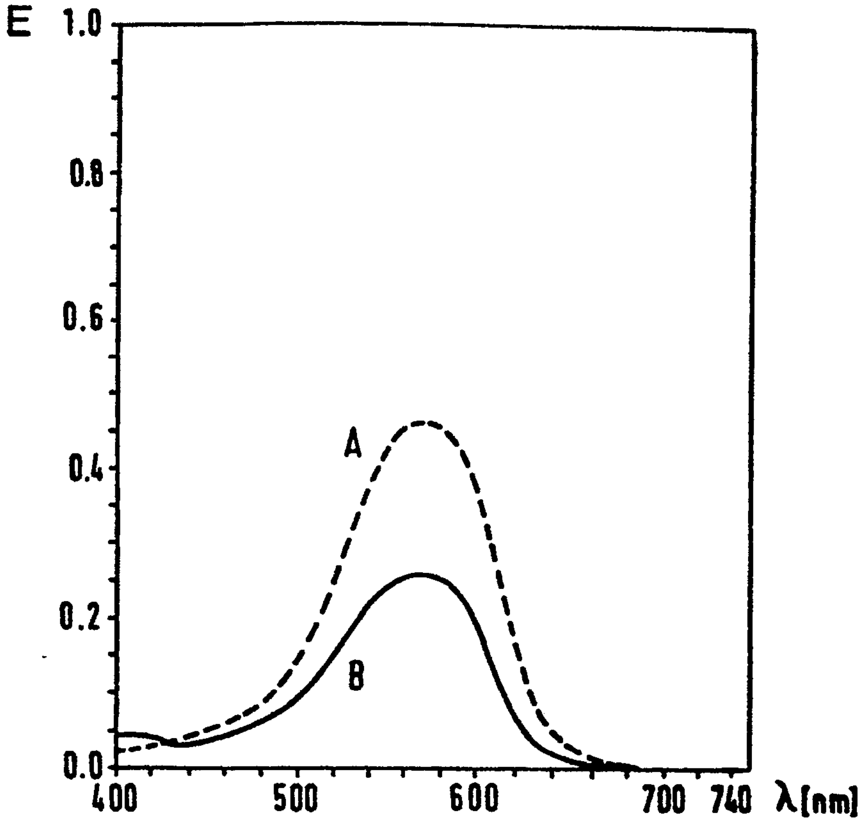
E
1.0
0.8
0.6
0.4
0.2
0.0
A
B
400
500
600
700
740
λ[nm]

FIG.2

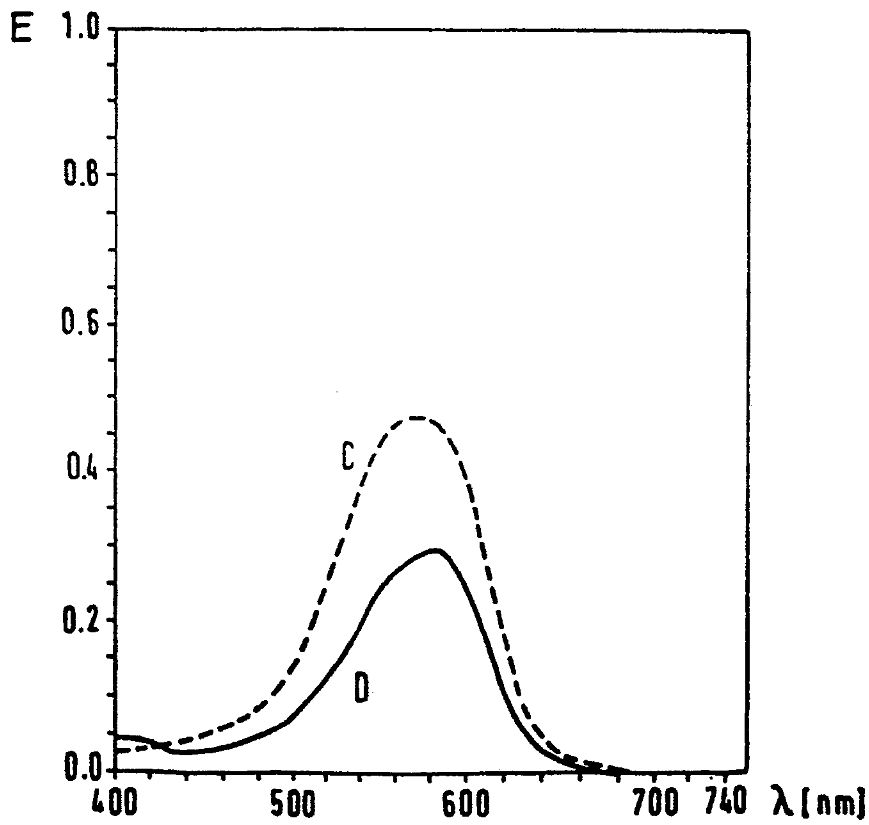
E
1.0
0.8
0.6
0.4
0.2
0.0
C
D
400
500
600
700
740
λ[nm]

FIG.3